# EUROPEAN PATENT APPLICATION

(11) **EP 1 665 992 A1**
(43) Date of publication of application: **07.06.2006**
(21) Application number: 05257130.4
(22) Date of filing: 18.11.2005
(51) Int. Cl.: A61B 17/15

(54) **Surgical instrument**

(30) Priority: 06.12.2004 GB 0426767
(71) Applicant: Biomet Uk Limited, Bridgend South Wales CF31 3XA (GB)
(72) Inventor: Murray, David Wycliffe, Oxford OX44 9HB (GB); Thomas, William Keith, Chippenham Wiltshire SN15 3XS (GB); Reeves, Edward Alexander, Gloucestershire GL11 4BT (GB)
(74) Representative: Giles, Ashley Simon

(57) **Abstract**

A surgical instrument 10 comprises a base member 12 adapted to be attached to a bone, a guide 14 adjustably connected to the base member, a first marker 34 removably attached to the base member and a second marker 36 removably attached to the guide. In navigated surgery, the first and second markers 34,36 enable the position of the guide 14 to be determined relative to the position of the base member 12 and bone, to which it is attached. The guide may have a planar cutting surface 15 for guiding a blade or a circular aperture (115, Fig 3) for guiding a drill.

## Description

This invention relates to a surgical instrument and particularly but not exclusively to an instrument for use in computer assisted surgery.

### Background

During most types of orthopaedic surgery it is necessary to resect specific areas of bone. The precision of resection is particularly important during joint replacement surgery, where it is necessary to make a cut or drill a hole in a precise location and orientation relative to a patient's bone. Traditional methods use mechanical tools to reference off a remote area of bone in order to determine the correct position in which to cut or drill. However, recent advances have produced an alternative to physical referencing in the form of navigated surgery.

In, for example, a knee replacement operation, a permanent marker, comprising an array of three reflective devices or light emitting diodes, is attached to the femur through an incision remote from the knee area. Two cameras are then used to capture the position of the marker, and hence the bone, in space. A probe, comprising a second marker, is then touched to specific areas of the femur, in order to capture each position in space relative to the first, fixed marker. In this way, a digital image of the surface of the bone is created. This image is displayed to the surgeon on a computer screen. During the subsequent operation, the first, fixed marker and cameras remain in place in order to provide a link between the actual bone and the digital image. Any physical movement of the bone is thus reflected on the computer screen. The computer is able to calculate, from the image of the bone, exactly where a cut or hole should be made and hence to indicate on the screen the required position of a cutting or drill guide. The appropriate guide is provided with a marker to enable the surgeon to observe the position of the guide relative to the bone on the computer screen. When the image of the guide is in the required position relative to the bone as viewed on the screen, the guide is fixed in place and the cut is made. A similar procedure is followed for resection of the tibia.

Using navigated surgery, it is possible to achieve greater accuracy in the cutting and drilling of bone. However, the procedures can be difficult to implement. Although the required position of the guide relative to the bone is indicated on the screen, in practise, it is extremely difficult to attach the guide in precisely the right position. The attachment procedure requires the drilling of holes through the bone into which bone screws are inserted to hold the guide in place. Once the holes are drilled, further, fine adjustment of the position of the guide is often not possible. Exact matching of the position and orientation of the guide with the ideal position indicated on the screen is therefore extremely difficult.

A further disadvantage of the procedure is the need for at least one extra incision in the leg, spaced from the operative incision, in order to attach a fixed marker to a bone. This extra incision increases the duration of the patient's post operation recovery time. The provision of the fixed marker also results in additional bone damage, caused by the screws that hold the marker in place.

### Summary of Invention

According to the present invention, there is provided a surgical instrument comprising a base member adapted to be attached to a bone, a guide adjustably connected to the base member, a first marker removably attached to the base member and a second marker removably attached to the guide, the first and second markers enabling the spatial position of the guide to be determined relative to the position of the base member.

It is an advantage of the invention that the surgical instrument provided is adapted to be attached to the bone within the space of a small incision, and by attaching the first marker to the base member and the second marker to the guide, it is not necessary to make any further incisions around the operating area in order to attach a fixed marker to a bone. This results in operations being less invasive than previously.

Preferably, the guide is provided with a guide surface for guiding a surgical tool.

Preferably, the instrument further comprises an adjustment means disposed between the base member and the guide to permit relative motion in three dimensions between the base member and the guide.

It is a further advantage of the invention that a surgeon is able to accurately position the guide, by virtue of its capability to be adjusted in three dimensions relative to the base member.

Preferably, the instrument also comprises a locking mechanism, suitable for locking the guide in a fixed position relative to the base member. The locking mechanism may comprise a single locking means, first and second locking means or first, second and third locking means.

Preferably, the adjustment means comprises a stem, having first and second ends. The first end of the stem may be slidably received within a recess extending through the base member. Preferably, the sliding connection is such that the distance of the second end of the stem from an adjacent surface of the base member may be adjusted. Preferably, the first locking means is provided on the base member adjacent the recess in order to lock the stem in a fixed position relative to the base member.

Preferably, the second end of the stem terminates in a substantially spherical ball. Preferably, the guide includes a socket such that the ball of the stem may be received in the socket to form a rotational "ball and socket" joint between the stem and the guide. It is an advantage of such a joint that the orientation of the guide relative to the base member may be adjusted in three dimensions. Preferably, the second locking means is provided on the rotational joint to lock the guide in a fixed orientation relative to the base member.

In a first, preferred embodiment, the guide has at least one planar surface for guiding a blade. The guide and base member of the first embodiment may be disposed in a substantially perpendicular relationship. The first embodiment of the invention may be suitable for resecting a first surface of a human tibia. Preferably, the base member of the instrument is substantially aligned with the long axis of the tibia and the planar surface of the guide is perpendicular to the long axis of the tibia.

In a second, alternative embodiment, the guide comprises first and second guide parts. Preferably, a sliding joint is disposed between the first and second guide parts to permit relative sliding motion between the first and second guide parts. Preferably, the third locking means is provided on the sliding joint to lock the fist and second guide parts in a fixed position relative to one another. Preferably, the guide includes an aperture therethrough suitable for guiding a drill bit. The aperture may be provided on the first or the second guide part.

Preferably, each of the first and second markers of the present invention comprises an array of a plurality of marking elements. The marking elements may comprise reflective elements or light emitting diodes.

According to another aspect of the present invention, there is provided a method of positioning a guide relative to a bone using the surgical instrument of the first aspect of the present invention, the method comprising the steps of:
a) attaching the base member of the instrument to the bone,
b) observing and recording the position of the base member in a computer by reading the position of the first marker,
c) recording in the computer the position of a plurality of points on the surface of the bone in order to identify the position of the bone relative to the position of the base member,
d) displaying an image of the bone on a computer screen,
e) indicating on the screen a desired location of the guide,
f) observing and recording the position of the guide relative to the base member and bone in the computer by reading the position of the second marker, and
g) moving the guide to the desired location and locking the adjusted position of the guide relative to the base member and bone.

According to yet another aspect of the present invention, there is provided a method of positioning a guide of a surgical instrument relative to a bone, the method comprising the steps of:
a) attaching a base member of the instrument to the bone,
b) observing and recording the position of the base member in a computer by reading the position of a first marker removably attached to the base member,
c) recording in the computer the position of a plurality of points on the surface of the bone in order to identify the position of the bone relative to the position of the base member,
d) displaying an image of the bone on a computer screen,
e) indicating on the screen a desired location of the guide,
f) observing and recording the position of the guide relative to the base member and bone in the computer by reading the position of a second marker removably attached to the guide, and
g) moving the guide to the desired location and locking the adjusted position of the guide relative to the base member and bone.

### Brief Description of Drawings

The invention will now be described by way of example only with reference to the accompanying drawings, in which:
Figure 1 is a diagrammatic perspective view of a first embodiment of a surgical instrument in accordance with the invention.
Figure 2 a plan view of the surgical instrument of Figure 1 attached to the upper part of a tibia for performing a partial resection of the tibial plateau.
Figure 3 is a diagrammatic plan view, partly in cross section, of a second embodiment of a surgical instrument in accordance with the invention.
Figure 4 is a plan view of the surgical instrument shown in Figure 3 attached to a femur for drilling a hole in a femoral condyle.

### Detailed Description of Preferred Embodiments

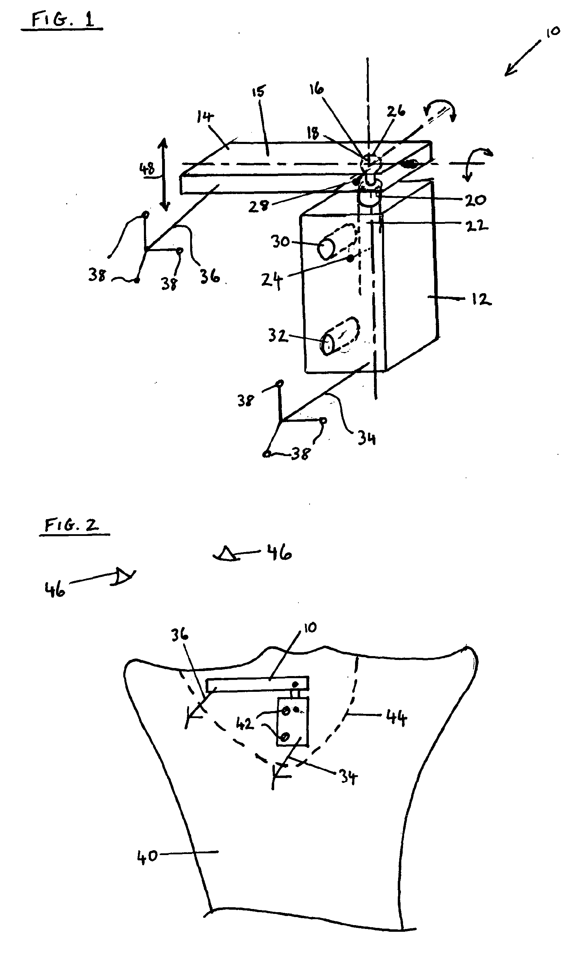

Referring firstly to Figure 1, a surgical instrument is indicated generally at 10. The surgical instrument 10 comprises a base member 12 and a cutting guide 14, adjustably connected together by means of a ball and socket joint 16. A planar guiding surface 15 is provided on the cutting guide 14, which, in use, guides a reciprocating saw or other cutting tool. A ball 18, of the joint 16, is mounted at the end of a stem 20, which is slidably received in an aperture 22 in the base member 12. A locking means 24, for example, a locking screw, is provided in the base member 12 for locking the position of the ball 18 and stem 20 relative to the base member 12.

The ball 18 is received in a socket 26 of the joint 16, provided in the cutting guide 14. A second locking means 28 is provided on the cutting guide 14, which locks the cutting guide relative to the ball 18 and stem 20. The position of the cutting guide 14 can therefore be locked relative to the position of the base member 12 by means of the first and second locking means 24,28 acting on the stem 20 and ball 18.

First and second apertures 30, 32 are provided through the base member 12, through which screws may be driven in order to secure the surgical instrument to a bone. A first marker 34 is removably attached to the base member 12, and a second marker 36 is removably attached to the cutting guide 14. The markers are those known in the art of digitised operative treatment or navigated surgery as arrays, and each comprises three spatially positioned light emitting diodes or reflectors 38, which can be identified by a camera or other suitable identifying means.

Referring now to figure 2, a standard surgical approach has been carried out for performing a partial knee replacement operation and the osteophytes have been removed. The instrument 10 is shown attached to a tibia 40 by means of a pair of screws 42, which are fastened to the tibia through the first and second apertures 30,32. The instrument is positioned against the tibia through a small incision in a patient's knee, which is indicated in dotted outline at 44. The screws are inserted into the tibia in the region of the tibial tubercle. At this point in the operation, the arrays 34,36 are removed from the instrument 10. A pair of cameras 46 capable of detecting the position of the arrays 34,36 are directed towards the operative area. The cameras 46 are linked to a computer and computer screen (not shown) in a known configuration.

The first array 34 is attached to the base member, which is attached to the tibia 40 and key points around the tibia are digitised in known manner, by means of a further array attached to a probe (not shown). These points typically include the medial and lateral malleolae; the centre of the tibial plateau; the surface of the posterior part of the medial tibial plateau (with the knee in maximal flexion) and the lateral side of the medial femoral condyle (with the knee in 90° flexion). Then, with the knee in full extension the patient's leg is moved around in order to identify the femoral head. The computer then determines the desired position of the saw cut across the top of the tibia 40. Typically, the plane of the cut will be perpendicular to the tibial axis in the coronal plane and sloping 7° in the saggital plane and 8 millimetres below the normal cartilage posteriorly, although the position of the cut may be adapted to suit a patient's anatomy.

The second array 36 is then attached to the cutting guide 14. The cutting guide 14 is then adjusted until the planar guiding surface 15 is in the correct orientation, which is determined by the surgeon with reference to the computer screen, where a digitised image of the tibia 40, guide 10 and ideal cut position are displayed. In order to adjust the instrument 10, the locking means 24,28 are released. This allows the cutting guide 14 to move relative to the base member 12, firstly in the direction of the axis of the stem 22, as indicated by arrow 48, and secondly in rotatational directions about perpendicular axes of the ball and socket joint 16 as indicated by the arrows 50,52. When the planar guiding surface 15 is correctly positioned, the locking means 24,28 are engaged to lock the cutting guide 14 in position relative to the base member 12 and the bone 40, to which the base member is attached. The necessary cut or cuts can then be made and the instrument removed by releasing the screws 42.

Optionally, the cutting guide 14 may have a further planar guiding surface (not shown) positioned substantially perpendicular to the planar guiding surface 15, for guiding a saw or other cutting implement in a vertical cut.

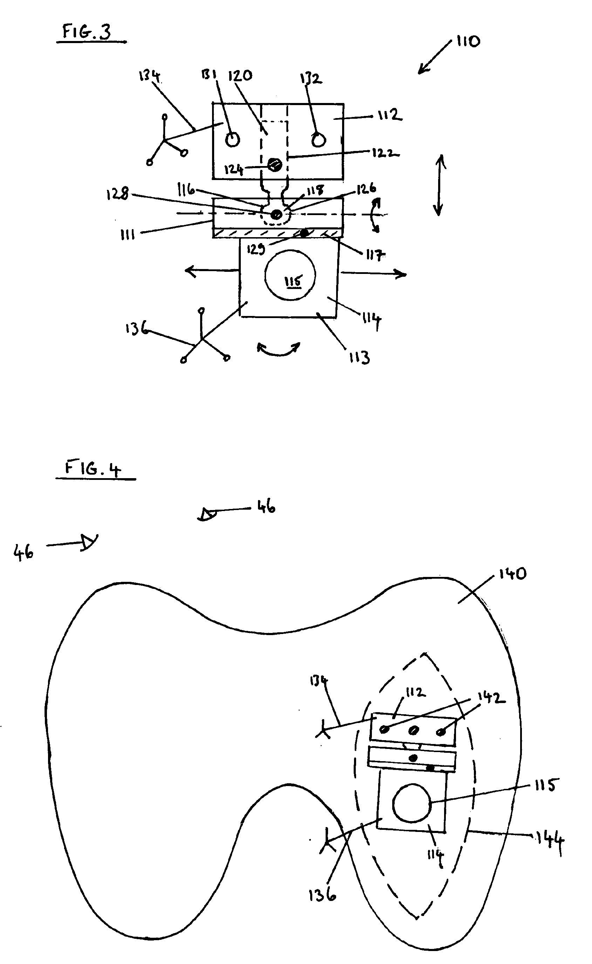

Referring now to Figure 3, a second embodiment of a surgical instrument is indicated generally at 110. The surgical instrument 110 comprises a base member 112 and a cutting guide 114, adjustably connected together by means of a ball and socket joint 116. The cutting guide 114 is provided in first and second parts 111,113. A circular hole 115 is provided through second part 113 of the cutting guide 114, which, in use, guides a drill, brooch or other cutting implement. A ball 118 of the joint 116 is mounted at the end of a stem 120, which is slidably received in an aperture 122 in the base member 112. A locking means 124, for example, a locking screw, is provided in the base member 112 for locking the position of the ball 118 and stem 120 relative to the base member 112.

The ball 118 is received in a socket 126 provided in the first part 111 of the cutting guide 14. A second locking means 128 is provided on the first part of the cutting guide 114, which locks the cutting guide relative to the ball 118 and stem 120. However, in this embodiment, a further degree of adjustability is provided by a sliding joint 117, which may be a dovetail slide, or any suitable sliding joint, provided between the first and second parts 111,113 of the cutting guide 114. A third locking means 129 is provided in one of the parts 111,113 of the cutting guide 114 for locking the position of the first part 111 relative to the second part 113 of the cutting guide. In this manner, the position of the cutting guide 114 can be locked relative to the position of the base member 112.

As in the first embodiment, first and second apertures 130, 132 are provided through the base member 112, through which screws may be driven in order to secure the surgical instrument to a bone. A first marker 134 is removably attached to the base member 112, and a second marker 136 is removably attached to the second part 113 of the cutting guide 114. The markers are those known in the art of digitised operative treatment as arrays as described previously.

The surgical instrument 110 is shown in use in Figure 4 during a partial knee replacement operation. The instrument is attached to a femur 140, by means of a pair of screws 142, which pass through the apertures 131, 132. The instrument 110, without the markers 134, 136, is positioned against the femur 140 through an incision, indicated in dotted outline at 144. The operating arrangement is as described with regard to the first embodiment, with cameras 46 linked to a computer and computer screen (not shown) in a known configuration.

The first marker or array 134, is attached to the base member 112, which is attached to the femur 140 and a series of points and planes are digitised in known manner, by means of a further array attached to a probe (not shown). In particular, the patient's leg is moved around in order to determine the longitudinal axis of the femur and the position of the centre of the femoral head. The patient's knee is angled at 90°, and the largest size possible of tibial measurement guide block (not shown) is placed in the flexion gap. The block is pushed against a vertical cut of the tibia, and a further array is attached to the block. The plane defined by the upper surface and the lateral surface of the block are captured by the cameras and computer. These planes define the vertical cut, the orientation of the horizontal cut and the position of the most posterior part of the femoral condyle.

The computer then determines the position of the hole to be drilled in the femur 140. This hole should point directly towards the centre of the femoral head. The distance of the axis of the hole above the posterior part of the femoral condyle is equal to the radius of the femoral component. The distance that the hole is offset from the plane of the vertical cut of the tibia, which is perpendicular to the horizontal cut is equal to half of the width of a meniscal bearing component plus 2.5 mm.

The second array 136 is then attached to the cutting guide 114, and the guide adjusted until it is in the correct orientation, which is determined by the surgeon with reference to the computer screen, where a digitised image is displayed. Once the guide 114 is locked in position by the locking means 124, 128, 129, the arrays 134, 136 are removed from the instrument 110 and the hole drilled in the femur, the drilling tool being guided in the hole 116 provided through the second part 113 of the cutting guide 114. The instrument 110 is then removed by removal of the screws 142.

Once removed, a posterior femoral saw guide (not shown) is inserted into the drilled hole in the femur 140. With the knee at 90° flexion and a valgus load applied to the tibia, the saw guide is aligned approximately parallel to the tibia. A pin is inserted in the upper hole of the guide and the posterior saw cut made. The saw guide is then removed. The operation is then completed in usual manner.

The embodiments 10, 110 of the surgical instrument shown, are typical arrangements of a surgical instrument within the scope of the claims, but it will be appreciated that other arrangements are possible which provide the required adjustability between the base members 12, 112 and the guide 14, 114 and fall within the scope of the claims.

## Claims

**1.** A surgical instrument comprising a base member adapted to be attached to a bone, a guide adjustably connected to the base member, a first marker removably attached to the base member and a second marker removably attached to the guide, the first and second markers enabling the position of the guide to be determined relative to the position of the base member.

**2.** A surgical instrument as claimed in claim 1, in which an adjustment means is disposed between the base member and the guide.

**3.** A surgical instrument as claimed in claim 1 or 2, in which a locking mechanism is provided suitable for locking the guide in a fixed position relative to the base member.

**4.** A surgical instrument as claimed in claim 3, in which the locking mechanism comprises first, second and third locking means.

**5.** A surgical instrument as claimed in any one of claims 2 to 4, in which the adjustment means includes a stem slidably received in an aperture extending through the base member.

**6.** A surgical instrument as claimed in claim 5, in which the first locking means is provided on the base member and is adapted to lock the stem in a fixed position relative to the base member.

**7.** A surgical instrument as claimed in claim 5 or 6, in which one end of the stem terminates in a ball.

**8.** A surgical instrument as claimed in any one of the preceding claims, in which the guide includes a socket.

**9.** A surgical instrument as claimed in claim 8, in which the ball is received in the socket to form a rotational joint between the stem and the guide.

**10.** A surgical instrument as claimed in claim 9, in which the second locking means is provided on the guide and is adapted to lock the ball of the stem in a fixed position relative to the guide.

**11.** A surgical instrument as claimed in any one of the preceding claims, in which the guide has at least one planar surface for guiding a blade.

**12.** A surgical instrument as claimed in any one of claims 1 to 10, in which the guide is comprised of first and second parts.

**13.** A surgical instrument as claimed in claim 12, in which a sliding joint is disposed between the first and second parts.

**14.** A surgical instrument as claimed in claim 13, in which the third locking means is provided on the first part and is adapted to lock the first and second parts of the guide together.

**15.** A surgical instrument as claimed in any one of claims 1 to 10 and 12 to 14, in which the guide has an aperture therethrough, suitable for guiding a drill bit.

**16.** A surgical instrument as claimed in claim 16, in which the aperture is provided on the second guide part.

**17.** A surgical instrument as claimed in any one of the preceding claims, in which each of the first and second markers comprises an array of a plurality of marking elements.

**18.** A surgical instrument as claimed in claim 19, in which the marking elements comprise reflective elements or light emitting diodes.

**21.** A surgical instrument as claimed in any preceding claim, in which the guide is provided with a guide surface for guiding a surgical tool.

**22.** A surgical instrument substantially as described herein with reference to and as illustrated in Figs 1-4 of the accompanying drawings.

**23.** A method of positioning a guide relative to a bone using a surgical instrument as claimed in any one of the preceding claims, the method comprising the steps of:
a) attaching the base member of the instrument to the bone,
b) observing and recording the position of the base member in a computer by reading the position of the first marker,
c) recording in the computer the position of a plurality of points on the surface of the bone in order to identify the position of the bone relative to the position of the base member,
d) displaying an image of the bone on a computer screen,
e) indicating on the screen a desired location of the guide,
f) observing and recording the position of the guide relative to the base member and bone in the computer by reading the position of the second marker, and
g) moving the guide to the desired location and locking the adjusted position of the guide relative to the base member and bone.
